(19) Europäisches Patentamt / European Patent Office / Office européen des brevets

(11) **EP 4 352 741 B1**

(12) **EUROPEAN PATENT SPECIFICATION**

(45) Date of publication and mention
of the grant of the patent:
**07.05.2025 Bulletin 2025/19**

(21) Application number: **22730930.9**

(22) Date of filing: **03.06.2022**

(51) International Patent Classification (IPC):
**G16H 40/60** (2018.01) **A61B 6/00** (2024.01)

(52) Cooperative Patent Classification (CPC):
**G16H 40/60; A61B 6/54; A61B 8/5261;**
A61B 8/4263

(86) International application number:
**PCT/EP2022/065148**

(87) International publication number:
**WO 2022/258502 (15.12.2022 Gazette 2022/50)**

(54) **ORIENTING AN X-RAY DEVICE BASED ON AN ULTRASOUND IMAGE**

AUSRICHTUNG EINER RÖNTGENVORRICHTUNG AUF BASIS EINES ULTRASCHALLBILDES

ORIENTATION D'UN DISPOSITIF À RAYONS X SUR LA BASE D'UNE IMAGE ULTRASONORE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.06.2021 PCT/EP2021/065630**

(43) Date of publication of application:
**17.04.2024 Bulletin 2024/16**

(73) Proprietor: **Brainlab AG
81829 München (DE)**

(72) Inventors:
• **STEINLE, Wolfgang
81829 Munich (DE)**
• **WEI, Wei
81829 Munich (DE)**

(74) Representative: **SSM Sandmair
Patentanwälte Rechtsanwalt
Partnerschaft mbB
Joseph-Wild-Straße 20
81829 München (DE)**

(56) References cited:
WO-A2-2019/234495      JP-A- 2014 079 441
JP-B2- 6 014 866        US-A1- 2009 118 614
US-A1- 2018 235 573     US-A1- 2020 281 556
US-B2- 6 856 827

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

The present invention relates to computer-implemented methods of determining a control parameter for controlling a medical imaging device and a computer-implemented method of training a learning algorithm to establish a relationship between the values of a plurality of operating parameters of a first medical imaging device and values of a plurality of control parameters of a second medical imaging device and , corresponding computer programs, a computer-readable storage medium storing such a program and a computer executing the program, as well as a medical system comprising an electronic data storage device and the aforementioned computer.

**TECHNICAL BACKGROUND**

Adjusting an x-ray device to the optimal position, orientation, and imaging parameter for a standard image is not an easy task. The purpose of this invention is to guide a motorized x-ray device to perform optimal and standard image capture from the same perspective as the perspective used for capturing an image with a 3D ultrasound imaging device. An object of the present invention the ultrasound image is used as a guidance for orienting the x-ray device to capture an x-ray image such that the at least substantially same anatomical structure is imaged by the x-ray device as the anatomical structure depicted in the ultrasound image.

US 2018/0235573 A1 discloses methods and systems for multi-modality imaging. In one embodiment, a method comprises: during an ultrasound scan of a patient, coaligning an ultrasound image received during the ultrasound scan with a three-dimensional (3D) image of the patient acquired with an imaging modality prior to the ultrasound scan; calculating an angle for an x-ray source based on position information in the 3D image to align the x-ray source with the ultrasound image; and adjusting a position of the x-ray source based on the calculated angle. In this way, the same internal views of a patient may be obtained with multiple modalities during an intervention with minimal user input.

JP 6 014866 B2 discloses an x-ray photography condition setting system capable of setting a photographing angle of proper x-ray photographing.

WO 2019/234495 discloses various approaches for focusing an ultrasound transducer having multiple transducer elements include causing the transducer elements to transmit ultrasound waves to a target region and measure reflections of the ultrasound waves off the target region; for each of at least some of the transducer elements, adjusting a parameter value associated with said each transducer element based at least in part on parameter values associated with multiple measuring transducer elements weighted by signal quality metrics associated with the reflections measured by the measuring transducer elements so as to improve an ultrasound focus at the target region.

US 2020/0281556 A1 discloses that for x-ray detector pose estimation, a machine-learned model is used to estimate locations of markers, including occluded or other non-visible markers, from an image. The locations of the markers, including the non-visible markers are used to determine the pose of the X-ray detector for aligning an X-ray tube with the X-ray detector.

The present invention can be used for procedures e.g. in connection with operating an x-ray device such as LoopX, a product of Brainlab AG.

Aspects of the present invention, examples and exemplary steps and their embodiments are disclosed in the following. Different exemplary features of the invention can be combined in accordance with the invention wherever technically expedient and feasible.

**EXEMPLARY SHORT DESCRIPTION OF THE INVENTION**

In the following, a short description of the specific features of the present invention is given which shall not be understood to limit the invention only to the features or a combination of the features described in this section.

The disclosed method encompasses acquiring an operating parameter such as an imaging depth of an ultrasound probe used for imaging an anatomical structure (e.g. an internal organ or bony tissue or cartilage) and using the operating parameter as a guidance for appropriately orienting an x-ray device to generate an image of the same anatomical structure from for example the at least substantially same perspective as the perspective used for generating the ultrasound image. In examples, this is accomplished by using a transformation matrix which is suitable to transform the operating parameter of the ultrasound device into a corresponding control parameter of the x-ray device, or by using an appropriately configured, specifically trained, learnable algorithm such an artificial intelligence algorithm for determining the control parameter of the x-ray device.

## GENERAL DESCRIPTION OF THE INVENTION

In this section, a description of the general features of the present invention is given for example by referring to possible embodiments of the invention.

In general, the invention reaches the aforementioned object by providing, in a first aspect, a computer-implemented medical method of determining a control parameter for controlling a medical imaging device. The method according to the first aspect comprises executing, on at least one processor of at least one computer, the following exemplary steps which are executed by the at least one processor.

In a (for example first) step of the method according to the first aspect, first imaging device operating parameter data is acquired which describes a value of an operating parameter of a first medical imaging device which is for example a three-dimensional imaging device. For example, the operating parameter of the first medical imaging device comprises a plurality of operating parameters and the control parameter of the second medical imaging device comprises a plurality of control parameters. For example, the parameter relationship data is or has been determined by determining a parameter relationship matrix being the result of solving, for example by applying a singular value decomposition method, a linear equation system linking a vector comprising entries representing the plurality of operating parameters to a vector comprising entries representing the plurality of control parameters.

In a (for example second) step of the method according to the first aspect, parameter relationship data is acquired which describes a relationship between the value of the operating parameter of the first medical imaging device and a value of a control parameter of a second medical imaging device. For example, the operating parameter of the first medical imaging device comprises a plurality of operating parameters and the control parameter of the second medical imaging device comprises a plurality of control parameters. For example, the parameter relationship data is or has been determined by inputting values of the plurality of operating parameters and values of the plurality of control parameters and imaging signal data describing an image generated by the first medical imaging device into a learning algorithm configured to establish a relationship between the values of the plurality of operating parameters and values of the plurality of control parameters. For example, the learning algorithm is or has been configured to establish the relationship between the values of the plurality of operating parameters and the values of the plurality of control parameters by inputting, into the learning algorithm, values of the plurality of operating parameters and values of the plurality of control parameters and imaging signal data describing an image generated by the first medical imaging device and determining a resulting loss function.

In a (for example third) exemplary step of the method according to the first aspect, second imaging device control parameter data is determined based on the first imaging device operating parameter data and the parameter relationship data, wherein the second imaging device control parameter data describes the value of the control parameter of a second medical imaging device.

In a second aspect, the invention relates to a computer-implemented method of training a learning algorithm to establish a relationship between the values of a plurality of operating parameters of a first medical imaging device and values of a plurality of control parameters of a second medical imaging device. The method according to the second aspect comprises executing, on at least one processor of at least one computer, the following exemplary steps which are executed by the at least one processor.

In a (for example first) step of the method according to the second aspect, first imaging device operating parameter data is input into the learning algorithm. The first imaging device operating parameter data describes for example values of the plurality of operating parameters. Furthermore, second imaging device control parameter data is input into the learning algorithm. The second imaging device control parameter data describes for example values of the plurality of control parameters. Furthermore, data describing an image generated by the first medical imaging device is input into the learning algorithm. For example, the data describing an image generated by the first medical imaging device is image data representing the image.

In a (for example second) step of the method according to the second aspect, parameter relationship data is determined which represents a resulting loss function. For example, the parameter relationship data is determined by the learning algorithm based on the first imaging device operating parameter data and the second imaging device control parameter data and the data describing an image generated by the first medical imaging device.

In a third aspect, the invention relates to a computer-implemented method of determining a control parameter for controlling a medical imaging device. The method according to the third aspect comprises executing, on at least one processor of at least one computer, the following exemplary steps which are executed by the at least one processor.

In a (for example first) step of the method according to the third aspect, first imaging device operating parameter data is acquired which describes a value of an operating parameter of a first medical imaging device.

In a (for example second) step of the method according to the third aspect, imaging signal data is acquired which represents an image generated by the first medical imaging device.

In a (for example third) step of the method according to the third aspect, second imaging device control parameter data is determined which describes a value of the control parameter of a second medical imaging device. The second

**EP 4 352 741 B1**

imaging device control parameter data is determined by inputting, into a learning algorithm configured to establish a relationship between the value of the operating parameter and the value of the control parameter, the first imaging device operating parameter data and the imaging signal data and determining a resulting loss function. The learning algorithm has been trained for example by executing the method according to the second aspect.

In the methods according to the first, second and third aspect, the learning algorithm is a regression network.

In an example of the methods according to the first, second and third aspect, the operating parameter is an orientation of the first medical imaging device in a navigation reference system.

In an example of the methods according to the first, second and third aspect, the control parameter of the second medical imaging device is an orientation of the second medical imaging device in the navigation reference system.

In an example of the methods according to the first, second and third aspect, the orientation of the first medical imaging device defines an imaging perspective of the first medical imaging device onto the anatomical structure. The perspective and/or orientation of the ultrasound probe (i.e. the first medical imaging device) is defined as a centre line of its imaging volume.

In an example of the methods according to the first, second and third aspect, the orientation of the second medical imaging device defines an imaging perspective of the second medical imaging device onto the anatomical structure.

In an example of the methods according to the first, second and third aspect, first imaging device orientation data is determined which describes an orientation of a marker array having a predetermined spatial relationship relative to the first medical imaging device. Furthermore, for example second imaging device orientation data is determined which describes an orientation of a marker array having a predetermined spatial relationship relative to the second medical imaging device. The second imaging device control parameter data is determined based on the first imaging device orientation data and the second imaging device orientation data. For example, the first imaging device orientation data and the second imaging device orientation data is determined by tracking, for example positionally tracking, the first and second medical imaging device, for example by tracking the marker arrays using a digital camera(an optical tracking system). The digital camera (optical tracking system) can be a still image camera or a video image camera. Alternatively or additionally, the first imaging device orientation data and the second imaging device orientation data is determined by video-tracking the first and second medical imaging device without using any marker attached to it or with markers (such as stickers or electromagnetic markers) positioned on the first and second medical imaging device, or by evaluating signals issued by internal position detectors of the first and second medical imaging device (such as joint sensors in a mechanical arm forming part of the first and/or second medical imaging device). For example, the second imaging device control parameter data is determined such that the orientation of the second medical imaging device corresponds to the orientation of the first medical imaging device. For example, the orientation of at least one of the first medical imaging device or the second medical imaging device is acquired from a medical robot having a predetermined spatial relationship to the at least one of the first medical imaging device and being navigated in the navigation reference system. For example, the robot comprises a movable articulable arm having an end effector for conducting a medical procedure.

In an example of the methods according to the first, second and third aspect, the operating parameter is a perspective of the first medical imaging device onto an anatomical structure of a patient and the control parameter is an imaging perspective of the second medical imaging device onto the anatomical structure and the second imaging device control parameter data is determined such that the imaging perspective of the second medical imaging device onto the anatomical structure is at least substantially the same as the imaging perspective of the first medical imaging device onto the anatomical structure.

In an example of the methods according to the first, second and third aspect, the first medical imaging device is an ultrasound imaging device and the second medical imaging device is an x-ray imaging device, for example a C-arm or a computed x-ray tomography device or a cone-beam computed x-ray tomography device.

In an example of the methods according to the first, second and third aspect, the operating parameter comprises at least one of

- values (e.g. colour and/or greyscale values) derived from a medical image (such as ultrasound ;
- a medical image itself;
- values (e.g. colour and/or greyscale values) derived from a raw image signal;
- an imaging depth of the first medical imaging device (e.g. as a float value in millimeters);
- an imaging power of the first medical imaging device (e.g. as a float value in dB);
- a dynamic range of the first medical imaging device (e.g. as a float value in dB);
- an imaging frequency of the first medical imaging device (e.g. 2 MHz, 3.5 MHz, 4 MHz, 5 MHz as a float value in MHz);
- information describing whether imaging is conducted by the first medical imaging device is operated in Doppler mode (on/off, represented as 1 or 0), for example in Pulsed Wave Doppler, Continuous Wave Doppler, Colour Doppler, Power Doppler, Vector Flow Imaging, in Harmonic Imaging, Elastography / Sheer Wave Imaging, Contrast Enhanced Ultrasound Imaging (CEUS).

4

In an example of the methods according to the first, second and third aspect, the control parameter comprises at least one of

- an imaging voltage or imaging current or imaging duration or imaging range (e.g. degrees covered, e.g. 180° or 360° or short scans) of the second medical imaging device;
- a metal artefact reduction parameter of the second medical imaging device for reconstructing a three-dimensional image (e.g.: on/off, e.g. image region-specific by specifying the coordinates of a 3d image region that contains the metal);
- a collimator setting of the second medical imaging device;
- a distance between an anatomical structure and the second medical imaging device (e.g. as a float value in millimeters);
- information whether scatter correction is being applied by the second medical imaging device (e.g. on/off);
- a range of imaging angles of the second medical imaging device;
- information describing a type of an X-ray filter of the second medical imaging device (e.g. as a type selected from a list, represented by integer value).

In a fourth aspect, the present invention relates to a program which, when running on a computer or when loaded onto a computer, causes the computer to perform the method steps of the method according to the first, second or third aspect. A computer program stored on a disc is a data file, and when the file is read out and transmitted it becomes a data stream for example in the form of a (physical, for example electrical, for example technically generated) signal. The signal wave is in one example a data carrier signal carrying the aforementioned computer program.

In a fourth aspect, the present invention relates to a for example non-transitory or non-volatile computer-readable program storage medium on which the program according to the fourth aspect is stored or a program storage medium on which data defining the model parameters and the architecture of a learning algorithm which has been trained by executing the method according to the second aspect are stored.

In a fifth aspect, the present invention relates to a data carrier signal carrying the program according to the fourth aspect and/ or a data carrier signal carrying data defining the model parameters and the architecture of a learning algorithm which has been trained by executing the method according to the second aspect. The signal can be implemented as the signal wave, for example as an electromagnetic carrier wave. For example, the signal, for example the signal wave is constituted to be transmitted via a computer network, for example LAN, WLAN, WAN, mobile network, for example the internet. For example, the signal, for example the signal wave, is constituted to be transmitted by optic or acoustic data transmission. The data carrier signal can be embodied by a (physical, for example electrical, for example technically generated) signal wave, for example a digital signal wave, such as an electromagnetic carrier wave carrying information which represents the program according to the second aspect, which for example comprises code means which are adapted to perform any or all of the steps of the method according to the first, second or third aspect.

In a sixth aspect, the present invention relates to a data stream which carries the program according to the fourth aspect and/or a data stream which carries data defining the model parameters and the architecture of a learning algorithm which has been trained by executing the method according to the second aspect.

In a seventh aspect, the present invention relates to at least one computer comprising at least one processor and a memory, wherein the program according to the fourth aspect is running on the at least one processor or loaded into the memory of the computer.

In an eighth aspect, the present invention relates to a system for determining a control parameter for controlling a medical imaging device, comprising:

a) the at least one computer according to the seventh aspect;
b) at least one electronic data storage device storing the parameter relationship data; and
c) the program storage medium according to the fourth aspect,

wherein the at least one computer is operably coupled to

- the at least one electronic data storage device for acquiring, from the at least one electronic data storage device, the first imaging device operating parameter data, and for storing, in the at least one electronic data storage device, at least the second imaging device control parameter data; and
- as far as the program causes the computer to perform the method steps of the method according to the third aspect, the program storage medium for acquiring, from the program storage medium, data defining model parameters and an architecture of the learning algorithm configured to establish a relationship between the value of the operating parameter and the value of the control parameter.

In a ninth aspect, the present invention relates to a system for controlling a medical imaging device, comprising:

a) the system according to the eighth aspect;
b) the first medical imaging device; and
c) the second medical imaging device,

wherein the at least one computer is operably coupled to

- the first medical imaging device for acquiring, from the first medical imaging device, the first imaging device operating data; and
- the second medical imaging device for issuing, to the second medical imaging device, a control signal for controlling the second medical imaging device according to the second imaging device control data.

In a tenth aspect, the present invention also relates to the use of the system according to the eighth or ninth aspect for for determining a control parameter for controlling a medical imaging device.

**DEFINITIONS**

In this section, definitions for specific terminology used in this disclosure are offered which also form part of the present disclosure.

The method in accordance with the invention is for example a computer implemented method. For example, all the steps or merely some of the steps (i.e. less than the total number of steps) of the method in accordance with the invention can be executed by a computer (for example, at least one computer). An embodiment of the computer implemented method is a use of the computer for performing a data processing method. An embodiment of the computer implemented method is a method concerning the operation of the computer such that the computer is operated to perform one, more or all steps of the method.

The computer for example comprises at least one processor and for example at least one memory in order to (technically) process the data, for example electronically and/or optically. The processor being for example made of a substance or composition which is a semiconductor, for example at least partly n- and/or p-doped semiconductor, for example at least one of II-, III-, IV-, V-, VI-semiconductor material, for example (doped) silicon and/or gallium arsenide. The calculating or determining steps described are for example performed by a computer. Determining steps or calculating steps are for example steps of determining data within the framework of the technical method, for example within the framework of a program. A computer is for example any kind of data processing device, for example electronic data processing device. A computer can be a device which is generally thought of as such, for example desktop PCs, notebooks, netbooks, etc., but can also be any programmable apparatus, such as for example a mobile phone or an embedded processor. A computer can for example comprise a system (network) of "sub-computers", wherein each sub-computer represents a computer in its own right. The term "computer" includes a cloud computer, for example a cloud server. The term computer includes a server resource. The term "cloud computer" includes a cloud computer system which for example comprises a system of at least one cloud computer and for example a plurality of operatively interconnected cloud computers such as a server farm. Such a cloud computer is preferably connected to a wide area network such as the world wide web (WWW) and located in a so-called cloud of computers which are all connected to the world wide web. Such an infrastructure is used for "cloud computing", which describes computation, software, data access and storage services which do not require the end user to know the physical location and/or configuration of the computer delivering a specific service. For example, the term "cloud" is used in this respect as a metaphor for the Internet (world wide web). For example, the cloud provides computing infrastructure as a service (IaaS). The cloud computer can function as a virtual host for an operating system and/or data processing application which is used to execute the method of the invention. The cloud computer is for example an elastic compute cloud (EC2) as provided by Amazon Web Services™. A computer for example comprises interfaces in order to receive or output data and/or perform an analogue-to-digital conversion. The data are for example data which represent physical properties and/or which are generated from technical signals. The technical signals are for example generated by means of (technical) detection devices (such as for example devices for detecting marker devices) and/or (technical) analytical devices (such as for example devices for performing (medical) imaging methods), wherein the technical signals are for example electrical or optical signals. The technical signals for example represent the data received or outputted by the computer. The computer is preferably operatively coupled to a display device which allows information outputted by the computer to be displayed, for example to a user. One example of a display device is a virtual reality device or an augmented reality device (also referred to as virtual reality glasses or augmented reality glasses) which can be used as "goggles" for navigating. A specific example of such augmented reality glasses is Google Glass (a trademark of Google, Inc.). An augmented reality device or a virtual reality device can be used both to input information into the computer by user interaction and to display information outputted by the computer. Another example of a display device would be a standard computer monitor comprising for example a liquid crystal display operatively coupled to the computer for receiving display control data from the computer for generating signals used to display image information content on the display device. A specific embodiment of such a computer monitor is a digital lightbox. An example of such a digital lightbox is Buzz®, a product of Brainlab AG. The monitor may also be the monitor of a portable, for example handheld, device such as a smart phone or personal digital assistant or digital media player.

The invention also relates to a computer program comprising instructions which, when on the program is executed by a computer, cause the computer to carry out the method or methods, for example, the steps of the method or methods, described herein and/or to a computer-readable storage medium (for example, a non-transitory computer-readable storage medium) on which the program is stored and/or to a computer comprising said program storage medium and/or to a (physical, for example electrical, for example technically generated) signal wave, for example a digital signal wave, such as an electromagnetic carrier wave carrying information which represents the program, for example the aforementioned program, which for example comprises code means which are adapted to perform any or all of the method steps described herein. The signal wave is in one example a data carrier signal carrying the aforementioned computer program. The invention also relates to a computer comprising at least one processor and/or the aforementioned computer-readable storage medium and for example a memory, wherein the program is executed by the processor.

Within the framework of the invention, computer program elements can be embodied by hardware and/or software (this includes firmware, resident software, micro-code, etc.). Within the framework of the invention, computer program elements can take the form of a computer program product which can be embodied by a computer-usable, for example computer-readable data storage medium comprising computer-usable, for example computer-readable program instructions, "code" or a "computer program" embodied in said data storage medium for use on or in connection with the instruction-executing system. Such a system can be a computer; a computer can be a data processing device comprising means for executing the computer program elements and/or the program in accordance with the invention, for example a data processing device comprising a digital processor (central processing unit or CPU) which executes the computer program elements, and optionally a volatile memory (for example a random access memory or RAM) for storing data used for and/or produced by executing the computer program elements. Within the framework of the present invention, a computer-usable, for example computer-readable data storage medium can be any data storage medium which can include, store, communicate, propagate or transport the program for use on or in connection with the instruction-executing system, apparatus or device. The computer-usable, for example computer-readable data storage medium can for example be, but is not limited to, an electronic, magnetic, optical, electromagnetic, infrared or semiconductor system, apparatus or device or a medium of propagation such as for example the Internet. The computer-usable or computer-readable data storage medium could even for example be paper or another suitable medium onto which the program is printed, since the program could be electronically captured, for example by optically scanning the paper or other suitable medium, and then compiled, interpreted or otherwise processed in a suitable manner. The data storage medium is preferably a non-volatile data storage medium. The computer program product and any software and/or hardware described here form the various means for performing the functions of the invention in the example embodiments. The computer and/or data processing device can for example include a guidance information device which includes means for outputting guidance information. The guidance information can be outputted, for example to a user, visually by a visual indicating means (for example, a monitor and/or a lamp) and/or acoustically by an acoustic indicating means (for example, a loudspeaker and/or a digital speech output device) and/or tactilely by a tactile indicating means (for example, a vibrating element or a vibration element incorporated into an instrument). For the purpose of this document, a computer is a technical computer which for example comprises technical, for example tangible components, for example mechanical and/or electronic components. Any device mentioned as such in this document is a technical and for example tangible device.

The expression "acquiring data" for example encompasses (within the framework of a computer implemented method) the scenario in which the data are determined by the computer implemented method or program. Determining data for example encompasses measuring physical quantities and transforming the measured values into data, for example digital data, and/or computing (and e.g. outputting) the data by means of a computer and for example within the framework of the method in accordance with the invention. A step of "determining" as described herein for example comprises or consists of issuing a command to perform the determination described herein. For example, the step comprises or consists of issuing a command to cause a computer, for example a remote computer, for example a remote server, for example in the cloud, to perform the determination. Alternatively or additionally, a step of "determination" as described herein for example comprises or consists of receiving the data resulting from the determination described herein, for example receiving the resulting data from the remote computer, for example from that remote computer which has been caused to perform the determination. The meaning of "acquiring data" also for example encompasses the scenario in which the data are received or retrieved by (e.g. input to) the computer implemented method or program, for example from another program, a previous method step or a data storage medium, for example for further processing by the computer implemented method or program. Generation of the data to be acquired may but need not be part of the method in accordance with the invention. The expression "acquiring data" can therefore also for example mean waiting to receive data and/or receiving the data. The received data can for example be inputted via an interface. The expression "acquiring data" can also mean that the computer implemented method or program performs steps in order to (actively) receive or retrieve the data from a data source, for instance a data storage medium (such as for example a ROM, RAM, database, hard drive, etc.), or via the interface (for instance, from another computer or a network). The data acquired by the disclosed method or device, respectively, may be acquired from a database located in a data storage device which is operably to a computer for data transfer between the database and the computer, for example from the database to the computer. The computer acquires the data for use as an input for steps of determining data. The determined data can be output again to the same or another database to be stored for later use. The database or database used for implementing the disclosed method can be located on network data storage device or a network server (for example, a cloud data storage device or a cloud server) or a local data storage device (such as a mass storage device operably connected to at least one computer executing the disclosed method). The data can be made "ready for use" by performing an additional step before the acquiring step. In accordance with this additional step, the data are generated in order to be acquired. The data are for example detected or captured (for example by an analytical device). Alternatively or additionally, the data are inputted in accordance with the additional step, for instance via interfaces. The data generated can for example be inputted (for instance into the computer). In accordance with the additional step (which precedes the acquiring step), the data can also be provided by performing the additional step of storing the data in a data storage medium (such as for example a ROM, RAM, CD and/or hard drive), such that they are ready for use within the framework of the method or program in accordance with the invention. The step of "acquiring data" can therefore also involve commanding a device to obtain and/or provide the data to be acquired. In particular, the acquiring step does not involve an invasive step which would represent a substantial physical interference with the body, requiring professional medical expertise to be carried out and entailing a substantial health risk even when carried out with the required professional care and expertise. In particular, the step of acquiring data, for example determining data, does not involve a surgical step and in particular does not involve a step of treating a human or animal body using surgery or therapy. In order to distinguish the different data used by the present method, the data are denoted (i.e. referred to) as "XY data" and the like and are defined in terms of the information which they describe, which is then preferably referred to as "XY information" and the like.

It is the function of a marker to be detected by a marker detection device (for example, a camera or an ultrasound receiver or analytical devices such as CT or MRI devices) in such a way that its spatial position (i.e. its spatial location and/or alignment) can be ascertained. The detection device is for example part of a navigation system. The markers can be active markers. An active marker can for example emit electromagnetic radiation and/or waves which can be in the infrared, visible and/or ultraviolet spectral range. A marker can also however be passive, i.e. can for example reflect electromagnetic radiation in the infrared, visible and/or ultraviolet spectral range or can block x-ray radiation. To this end, the marker can be provided with a surface which has corresponding reflective properties or can be made of metal in order to block the x-ray radiation. It is also possible for a marker to reflect and/or emit electromagnetic radiation and/or waves in the radio frequency range or at ultrasound wavelengths. A marker preferably has a spherical and/or spheroid shape and can therefore be referred to as a marker sphere; markers can however also exhibit a cornered, for example cubic, shape.

## BRIEF DESCRIPTION OF THE DRAWINGS

In the following, the invention is described with reference to the appended figures which give background explanations and represent specific embodiments of the invention. The scope of the invention is however not limited to the specific features disclosed in the context of the figures, wherein

Fig. 1          illustrates the basic steps of the method according to the first aspect;
Fig. 2          illustrates the basic steps of the method according to the second aspect;
Fig. 3          illustrates the basic steps of the method according to the third aspect;
Figs. 4 to 6    illustrates embodiments of the methods according to the second and third aspect;
Figs. 7 and 8   show an application of the method according to the first and third aspect; and
Fig. 9          is a schematic illustration of the system according to the ninth aspect.

## DESCRIPTION OF EMBODIMENTS

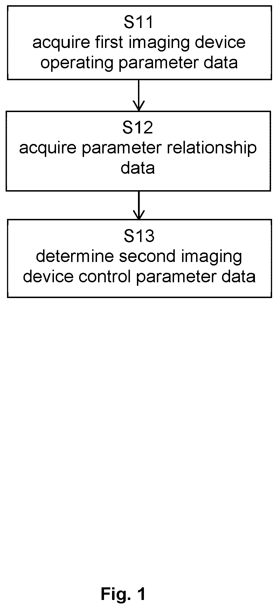

Figure 1 illustrates the basic steps of the method according to the first aspect, in which step S11 encompasses acquisition of the first imaging device operating parameter data, step S12 encompasses acquisition of the parameter relationship data and subsequent step S13 encompasses determination of the second imaging device control parameter data.

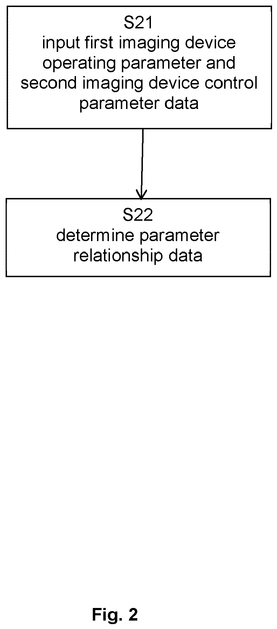

Figure 2 illustrates the basic steps of the method according to the second aspect, in which step S21 encompasses inputting the first imaging device operating parameter data, and subsequent step S22 encompasses determination of the parameter relationship data.

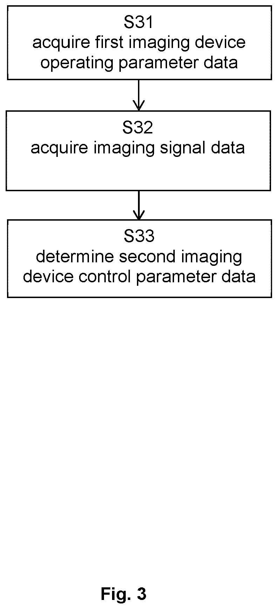

Figure 3 illustrates the basic steps of the method according to the third aspect, in which step S31 encompasses acquisition of the first imaging device operating parameter data, step S32 encompasses acquisition imaging signal data and subsequent step S33 encompasses determination of the second imaging device control parameter data.

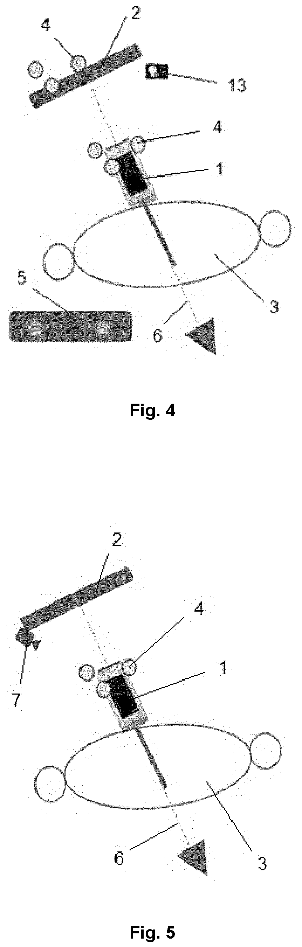

Figures 4 to 6 illustrate different embodiments of determining the orientation of the first and second medical imaging device, wherein the same reference signs denote the same entities throughout those figures.

Figure 4 shows an embodiment in which an ultrasound imaging device 1 representing the first medical imaging device is used to image an anatomical structure 3. An array of markers 4 is attached to the ultrasound imaging device 1 in a predetermined and for example fixed and known spatial relationship. The second medical imaging device is embodied by an x-ray imaging device 2 to which also an array of markers 4 is attached to the ultrasound imaging device 1 in a predetermined and for example fixed and known spatial relationship. A network device interface (NDI) camera 5 is used to track the position of the marker arrays and on the basis of the result of the tracking to determine the orientation of the x-ray imaging device 2 and the ultrasound imaging device 1 for generating an image of the anatomical structure 3 which is output to a user on a screen 13. A control signal for controlling the x-ray imaging device 2 to attain an orientation relative to the anatomical structure 3 which is at least substantially the same as the orientation 6 of the ultrasound imaging device 2 relative to the anatomical structure 3 is then determined. In one example, the x-ray imaging device 2 is controlled accordingly and for example moved into the respective orientation.

Figure 5 shows an embodiment which is similar to the embodiment of Figure 4. The main difference is that the ultrasound imaging device 1 is tracked using a calibrated detector camera 7 which has a predetermined and for example fixed and known spatial relationship to the x-ray imaging device 2. For example, the calibrated detector camera 4 is fastened to the x-ray imaging device 2. The functionality of the embodiment of Figure 5 otherwise works in the same way as the functionality of the embodiment of Figure 4.

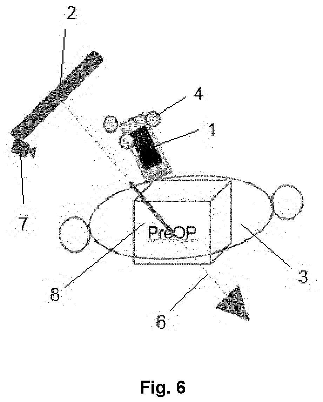

Figure 6 shows an embodiment which is similar to the embodiment of Figure 4. The main difference is that the ultrasound imaging device 1 is tracked using a video camera 7 which has a predetermined and for example fixed and known spatial relationship to the x-ray imaging device 2. Also, a pre-operative data set 8 showing a three-dimensional image (tomographic image) of the anatomical structure 3 and a plan for conducting a medical procedure are additionally used for orienting the x-ray imaging device 2 to generate the desired view of the anatomical structure 3 in a medical image. For example, the video camera 4 is fastened to the x-ray imaging device 2. The functionality of the embodiment of Figure 5 otherwise works in the same way as the functionality of the embodiment of Figure 4.

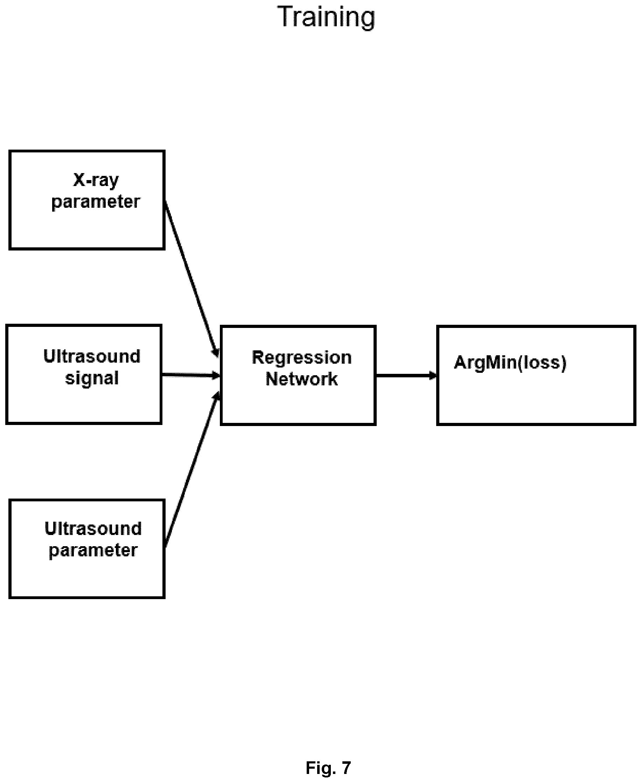

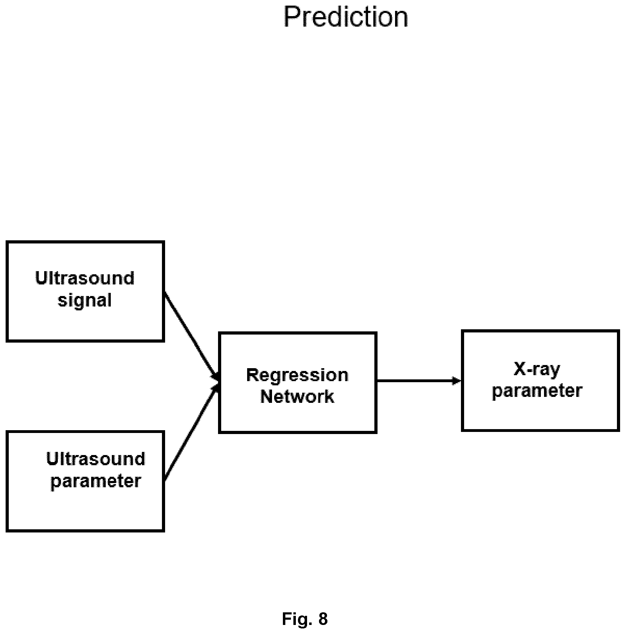

Figure 7 shows an embodiment of the method according to the second aspect in which an x-ray parameter representing the first imaging device operating parameter data is input, into a regression network representing the learning algorithm, together with an ultrasound signal representing the imaging signal data and an ultrasound parameter representing the second imaging device control parameter data which is known from a look-up table. The result of processing these data sets by the regression network is a loss function ArgMin(loss). The training phase takes the ultrasound signal, ultrasound parameters and ground truth x-ray parameters as input and minimizes the loss function. The loss function represents the difference between predicted and the ground truth x-ray parameters. In the prediction phase (i.e. when using the trained network), the regression network can predict x-ray parameters from an input ultrasound signal and an image, as illustrated by Figure 8.

Benefit of the machine learning approach over the manual (look-up table) approach is that we do not necessarily need to pre-process the image data or select important parameters or features.

An artificial intelligence-based regression network (e.g. ResNet and/or DenseNet) and/or a look-up table approach can be trained with ultrasound parameters and/or images as input and x-ray parameters as output. The regression network combines the ultrasound and x-ray parameters in a non-linear way.

The look-up table can take the form of a system of linear equations which establishes the relationship between the value of the operating parameter of the first medical imaging device and the value of the control parameter of the second medical imaging device, and therefore is an embodiment of the parameter relationship data.

A model of the relationship is established as $\overrightarrow{X_n} = C_{n \times m} \cdot \overrightarrow{U_m}$. $C_{n \times m}$ represents the parameter matrix which describes relation between ultrasound parameter vector $\overrightarrow{U_m}$ and an x-ray parameter vector $\overrightarrow{X_n}$. The coefficients $C_{ij}$ (with $i = 1, ..., n$ and $j = 1, ..., m$) can be derived by solving equation system using a singular value decomposition (SVD) method. $C_{ij}$ are double or float numbers, $X_i$ and $U_i$ are double or float numbers.

Let in an example $n = 2$ and $m = 3$.

$$\begin{pmatrix} X_1 \\ X_2 \end{pmatrix} = \begin{pmatrix} C_{11} C_{12} C_{13} \\ C_{21} C_{22} C_{23} \end{pmatrix} \begin{pmatrix} U_1 \\ U_2 \\ U_3 \end{pmatrix} \tag{1}$$

$$\Rightarrow \quad X_1 = C_{11} \cdot U_1 + C_{12} \cdot U_2 + C_{13} \cdot U_3 + C_{21} \cdot 0 + C_{22} \cdot 0 + C_{23} \cdot 0$$

$$\Rightarrow \quad X_2 = C_{11} \cdot 0 + C_{12} \cdot 0 + C_{13} \cdot 0 + C_{21} \cdot U_1 + C_{22} \cdot U_2 + C_{23} \cdot U_3$$

$$\Rightarrow \quad \begin{pmatrix} X_1 \\ X_2 \end{pmatrix} = \begin{pmatrix} U_1 \ U_2 \ U_3 \ 0 \ 0 \ 0 \\ 0 \ 0 \ 0 \ U_1 \ U_2 \ U_3 \end{pmatrix} \begin{pmatrix} C_{11} \\ C_{12} \\ C_{13} \\ C_{21} \\ C_{22} \\ C_{23} \end{pmatrix}$$

$$\Rightarrow \quad \begin{pmatrix} U_1 \ U_2 \ U_3 \ 0 \ 0 \ 0 \\ 0 \ 0 \ 0 \ U_1 \ U_2 \ U_3 \end{pmatrix} \begin{pmatrix} C_{11} \\ C_{12} \\ C_{13} \\ C_{21} \\ C_{22} \\ C_{23} \end{pmatrix} - \begin{pmatrix} X_1 \\ X_2 \end{pmatrix} = 0$$

$$(2)$$

$$\Rightarrow \quad \begin{pmatrix} U_1 \ U_2 \ U_3 \ 0 \ 0 \ 0 - X_1 \\ 0 \ 0 \ 0 \ U_1 \ U_2 \ U_3 - X_2 \end{pmatrix} \begin{pmatrix} C_{11} \\ C_{12} \\ C_{13} \\ C_{21} \\ C_{22} \\ C_{23} \\ 1 \end{pmatrix} = 0$$

$$\begin{pmatrix} U_1 \ U_2 \ U_3 \ 0 \ 0 \ 0 - X_1 \\ 0 \ 0 \ 0 \ U_1 \ U_2 \ U_3 - X_2 \end{pmatrix} := A$$

$C_{ij}$ are the wanted parameters and can be calculated by computing SVD(A) and selecting an eigenvector $\vec{v}$ which coincides with the minimum eigenvalue E and normalizing $\vec{v}$ by its last element.

The present invention seeks to guide an x-ray imaging device using a 3D ultrasound imaging device. A user can sweep the patient using the ultrasound imaging device and target the anatomical structure with centre line in the ultrasound volume. The relative position between the x-ray and ultrasound imaging devices can be derived by three different methods:

- tracking by NDI camera
- tracking by calibrated detector camera
- get the position using a surgical robot and fixed ultrasound calibration plate on the x-ray detector.

Once the position is known, the X-Ray device can be adjusted to the position in which the central x-ray beam is aligned with the centre line of the ultrasound volume. In addition, pre-operative data can be aligned with the ultrasound volume. In this case the central x-ray beam can be aligned to the predefined trajectory in the pre-operative data. In addition to the trajectory optimization, x-ray scan trajectories can be optimized with the ultrasound device. An ultrasound sweep can be used to define the anatomical structure that the user wants to see in the imaging volume or the region that should not be scanned by the x-ray imaging device. Based on those predefinitions coming from the ultrasound image, the x-ray imaging device can optimize its collimator, scan trajectories for a precise 3D scan. In addition, implants can be recognized and used for the artefact reduction.

As values derived from an image, multiple values are entered as separate $U_i$ into the matrix equations:

- statistical values: Image entropy, values derived from histogram (min, max, mean, median) or histogram itself

- PCA of image https://medium.com/@sebastiannorena/pca-principal-components-analysis-applied-to-images-of-faces-d2fc2c083371
- position, shape and type (tissue class) of segmented objects or main tissue class(es) of image
- Fourier spectrum: FFT of image

**Image itself:

Also, the image itself can be transformed into a list of values (e.g. after downsampling). Each value from the list is entered as a single parameter $U_i$ into the equations.

Manual training during production/calibration for the look-up table use case can be achieved e.g. by imaging a phantom and conducting the following steps:

1. determining a set of ultrasound parameters $U_i$, and optionally acquiring images
2. determining a set of optimal x-ray parameters $X_i$ to achieve an optimal x-ray image
3. repeating steps 1 and 2 N times, e.g. N = 4 for the example above (depending on the number of $C_{ij}$)
4. constructing matrix A of lines in equation (2) above and solving it for $C_{ij}$
5. storing $C_{ij}$ in the system;

The system uses e.g. equation (1) and determines optimal X-ray parameters $X_i$.

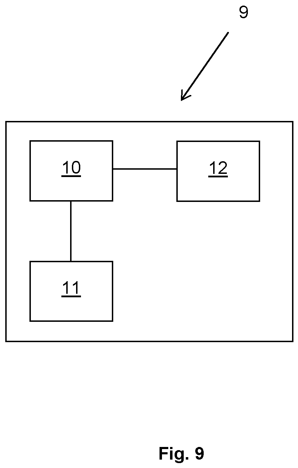

Figure 9 is a schematic illustration of the system 10 according to the fifth aspect. The system is in its entirety identified by reference sign 10 and comprises a computer 11, an electronic data storage device (such as a hard disc) 13 for storing at least the parameter relationship data and a program storage medium 12 storing the program according to the fourth aspect. The components of the system 10 have the functionalities and properties explained above with regard to the eighth aspect of this disclosure.

## Claims

1. A computer-implemented method of determining a control parameter for controlling a medical imaging device, the method comprising the following steps:

   a) first imaging device operating parameter data is acquired (S11) which describes a value of an operating parameter of a first medical imaging device (1), wherein the operating parameter is an orientation of the first medical imaging device (1) relative to an anatomical structure in a navigation reference system;
   b) parameter relationship data is acquired (S12) which describes a relationship between the value of the operating parameter of the first medical imaging device (1) and a value of a control parameter of a second medical imaging device (2), wherein the control parameter of the second medical imaging device (2) is an orientation of the second medical imaging device (2) relative to the anatomical structure in the navigation reference system;
   c) second imaging device control parameter data is determined (S13) based on the first imaging device operating parameter data and the parameter relationship data, wherein the second imaging device control parameter data describes the value of the control parameter of the second medical imaging device (2);

   first imaging device orientation data is determined by tracking the first medical imaging device; and
   second imaging device orientation data is determined by tracking the second medical imaging device, **characterised in that** the second imaging device control parameter data is determined based on the first imaging device orientation data and the second imaging device orientation data such that the orientation of the second medical imaging device (2) corresponds to the orientation of the first medical imaging device (1).

2. The method according to the preceding claim, wherein the operating parameter of the first medical imaging device (1) comprises a plurality of operating parameters and the control parameter of the second medical imaging device (2) comprises a plurality of control parameters and the parameter relationship data is or has been determined by determining a parameter relationship matrix being the result of solving, for example by applying a singular value decomposition method, a linear equation system linking a vector comprising entries representing the plurality of operating parameters to a vector comprising entries representing the plurality of control parameters.

3. The method according to claim 1, wherein the operating parameter of the first medical imaging device (1) comprises a plurality of operating parameters and the control parameter of the second medical imaging device (2) comprises a plurality of control parameters and the parameter relationship data is or has been determined by inputting values of the plurality of operating parameters and values of the plurality of control parameters and imaging signal data describing an image generated by the first medical imaging device into a learning algorithm configured to establish a relationship between the values of the plurality of operating parameters and values of the plurality of control parameters.

4.  The method according to the preceding claim, wherein the learning algorithm is or has been configured to establish the relationship between the values of the plurality of operating parameters and the values of the plurality of control parameters by inputting, into the learning algorithm, values of the plurality of operating parameters and values of the plurality of control parameters and imaging signal data describing an image generated by the first medical imaging device (1) and determining a resulting loss function.

5.  A computer-implemented method of training a learning algorithm to establish a relationship between the values of a plurality of operating parameters of a first medical imaging device (1), wherein the operating parameter is an orientation of the first medical imaging device (1) relative to an anatomical structure in a navigation reference system and values of a plurality of control parameters of a second medical imaging device (2), wherein the control parameter of the second medical imaging device (2) is an orientation of the second medical imaging device (2) relative to the anatomical structure in the navigation reference system, the method comprising:

    inputting (S21), into the learning algorithm, first imaging device operating parameter data describing values of the plurality of operating parameters and second imaging device control parameter data describing values of the plurality of control parameters and data describing an image generated by the first medical imaging device (1), wherein

    first imaging device orientation data is determined by tracking the first medical imaging device; and
    second imaging device orientation data is determined by tracking the second medical imaging device,

    **characterised in that** the second imaging device control parameter data is determined based on the first imaging device orientation data and the second imaging device orientation data such that the orientation of the second medical imaging device (2) corresponds to the orientation of the first medical imaging device (1); and by determining (S22) parameter relationship data by minimizing a loss function.

6.  A computer-implemented method of determining a control parameter for controlling a medical imaging device, the method comprising the following steps:

    a) first imaging device operating parameter data is acquired (S31) which describes a value of an operating parameter of a first medical imaging device (1), wherein the operating parameter is an orientation of the first medical imaging device (1) relative to an anatomical structure in a navigation reference system;
    b) imaging signal data is acquired (S32) which represents an image generated by the first medical imaging device (1);
    c) second imaging device control parameter data describing a value of the control parameter of a second medical imaging device (2) is determined (S33), wherein the control parameter of the second medical imaging device (2) is an orientation of the second medical imaging device (2) relative to the anatomical structure in the navigation reference system, **characterised in that** the second imaging device control parameter is determined such that the orientation of the second medical imaging device (2) corresponds to the orientation of the first medical imaging device (1) by inputting, into a learning algorithm configured to establish a relationship between the value of the operating parameter and the value of the control parameter which has been trained for example by executing the method according to the preceding claim, the first imaging device operating parameter data and the imaging signal data and determining a resulting loss function.

7.  The method according to any one of the preceding claims, wherein

    the orientation of the first medical imaging device (1) defines an imaging perspective of the first medical imaging device (1) onto an anatomical structure (3) and
    the orientation of the second medical imaging device (2) defines an imaging perspective of the second medical imaging device (2) onto the anatomical structure (3).

8.  The method according to any one of the preceding claims, wherein

the first medical imaging device (1) is an ultrasound imaging device and

the second medical imaging device (2) is an x-ray imaging device, for example a C-arm.

9. A program which, when running on a computer (10) or when loaded onto a computer (10), causes the computer (10) to perform the method steps of the method according to any one of the preceding claims.

10. A program storage medium (11) on which the program according to the preceding claim is stored or a program storage medium (11) on which data defining the model parameters and the architecture of a learning algorithm which has been trained by executing the method according to claim 5 are stored.

11. A data carrier signal carrying the program according to claim 9, and/ or a data carrier signal carrying data defining the model parameters and the architecture of a learning algorithm which has been trained by executing the method according to claim 5.

12. At least one computer (10) comprising at least one processor and a memory, wherein the program according to claim 9 is running on the at least one processor or loaded into the memory of the computer (10).

13. A system (9) for determining a control parameter for controlling a medical imaging device, comprising:

a) the at least one computer (10) according to the preceding claim;
b) at least one electronic data storage device (12) storing the parameter relationship data; and
c) the program storage medium (11) according to claim 10,

wherein the at least one computer (10) is operably coupled to

- the at least one electronic data storage device (12) for acquiring, from the at least one electronic data storage device (12), the first imaging device operating parameter data, and for storing, in the at least one electronic data storage device (12), at least the second imaging device control parameter data; and
- as far as the program causes the computer (10) to perform the method steps of the method according to claim 6, the program storage medium (11) for acquiring, from the program storage medium (11), data defining model parameters and an architecture of the learning algorithm configured to establish a relationship between the value of the operating parameter and the value of the control parameter.

14. A system for controlling a medical imaging device, comprising:

a) the system (9) according to the preceding claim;
b) the first medical imaging device (1); and
c) the second medical imaging device (2),

wherein the at least one computer (10) is operably coupled to

- the first medical imaging device (1) for acquiring, from the first medical imaging device (1), the first imaging device operating data; and
- the second medical imaging device (2) for issuing, to the second medical imaging device (2), a control signal for controlling the second medical imaging device according to the second imaging device control data.

**Patentansprüche**

1. Computerimplementiertes Verfahren zur Bestimmung eines Steuerparameters zur Steuerung einer medizinischen Bildgebungsvorrichtung, wobei das Verfahren die folgenden Schritte umfasst:

a) erste Bildgebungsvorrichtungsbetriebsparameterdaten werden erfasst (S11), die einen Wert eines Betriebsparameters eines ersten medizinischen Bildgebungsgeräts (1) beschreiben, wobei der Betriebsparameter eine Ausrichtung des ersten medizinischen Bildgebungsgeräts (1) relativ zu einer anatomischen Struktur in einem Navigationsbezugsystem ist;
b) Parameterbeziehungsdaten werden erfasst (S12), die eine Beziehung zwischen dem Wert des Betriebsparameters des ersten medizinischen Bildgebungsgeräts (1) und einem Wert eines Steuerparameters eines zweiten medizinischen Bildgebungsgeräts (2) beschreiben, wobei der Steuerparameter des zweiten medizinischen Bildgebungsgeräts (2) eine Ausrichtung des zweiten medizinischen Bildgebungsgeräts (2) relativ zu der anatomischen Struktur in dem Navigationsbezugsystem ist;

c) zweite Bildgebungsvorrichtungssteuerparameterdaten werden auf der Grundlage der ersten Bildgebungsvorrichtungsbetriebsparameterdaten und der Parameterbeziehungsdaten bestimmt (S13), wobei die zweiten Bildgebungsvorrichtungs-Steuerparameterdaten den Wert des Steuerparameters der zweiten medizinischen Bildgebungsvorrichtung (2) beschreiben;

erste Bildgebungsvorrichtungsausrichtungsdaten werden durch Tracking der ersten medizinischen Bildgebungsvorrichtung bestimmt; und

zweite Bildgebungsvorrichtungsausrichtungsdaten werden durch Tracking der zweiten medizinischen Bildgebungsvorrichtung ermittelt,

**dadurch gekennzeichnet, dass** die zweiten Bildgebungsvorrichtungssteuerparameterdaten auf der Grundlage der ersten Bildgebungsvorrichtungsausrichtungsdaten und der zweiten Bildgebungsvorrichtungsausrichtungsdaten so bestimmt werden, dass die Ausrichtung der zweiten medizinischen Bildgebungsvorrichtung (2) der Ausrichtung der ersten medizinischen Bildgebungsvorrichtung (1) entspricht.

2. Verfahren nach dem vorhergehenden Anspruch, wobei der Betriebsparameter der ersten medizinischen Bildgebungsvorrichtung (1) eine Vielzahl von Betriebsparametern umfasst und der Steuerparameter der zweiten medizinischen Bildgebungsvorrichtung (2) eine Vielzahl von Steuerparametern umfasst und die Parameterbeziehungsdaten durch Bestimmen einer Parameterbeziehungsmatrix bestimmt werden oder wurden, die das Ergebnis des Lösens eines linearen Gleichungssystems ist, das einen Vektor, der Einträge umfasst, die die Vielzahl von Betriebsparametern darstellen, mit einem Vektor verknüpft, der Einträge umfasst, die die Vielzahl von Steuerparametern darstellen, z.B. durch Anwenden eines Singulärwertzerlegungsverfahrens.

3. Verfahren nach Anspruch 1, wobei der Betriebsparameter der ersten medizinischen Bildgebungsvorrichtung (1) eine Vielzahl von Betriebsparametern umfasst und der Steuerparameter der zweiten medizinischen Bildgebungsvorrichtung (2) eine Vielzahl von Steuerparametern umfasst und die Parameterbeziehungsdaten durch Eingabe von Werten der Vielzahl von Betriebsparametern und Werten der Vielzahl von Steuerparametern und Bildsignaldaten, die ein von der ersten medizinischen Bildgebungsvorrichtung erzeugtes Bild beschreiben, in einem Lernalgorithmus bestimmt werden oder wurden, der so konfiguriert ist, dass er eine Beziehung zwischen den Werten der Vielzahl von Betriebsparametern und Werten der Vielzahl von Steuerparametern herstellt.

4. Verfahren nach dem vorhergehenden Anspruch, wobei der Lernalgorithmus so konfiguriert ist oder wurde, dass er die Beziehung zwischen den Werten der mehreren Betriebsparameter und den Werten der mehreren Steuerparameter herstellt, indem er Werte der mehreren Betriebsparameter und Werte der mehreren Steuerparameter und Bildsignaldaten, die ein von der ersten medizinischen Bildgebungsvorrichtung (1) erzeugtes Bild beschreiben, in den Lernalgorithmus eingibt und eine resultierende Verlustfunktion bestimmt.

5. Computerimplementiertes Verfahren zum Trainieren eines Lernalgorithmus, um eine Beziehung zwischen den Werten einer Vielzahl von Betriebsparametern einer ersten medizinischen Bildgebungsvorrichtung (1), wobei der Betriebsparameter eine Ausrichtung der ersten medizinischen Bildgebungsvorrichtung (1) relativ zu einer anatomischen Struktur in einem Navigationsbezugsystem ist, und Werten einer Vielzahl von Steuerparametern einer zweiten medizinischen Bildgebungsvorrichtung (2) herzustellen, wobei der Steuerparameter der zweiten medizinischen Bildgebungsvorrichtung (2) eine Ausrichtung der zweiten medizinischen Bildgebungsvorrichtung (2) relativ zu der anatomischen Struktur in dem Navigationsbezugsystem ist, wobei das Verfahren umfasst:

Eingeben (S21) von ersten Bildgebungsvorrichtungsbetriebsparameterdaten, die Werte der Vielzahl von Betriebsparametern beschreiben, und von zweiten Bildgebungsvorrichtungssteuerparameterdaten, die Werte der Vielzahl von Steuerparametern beschreiben, und von Daten, die ein von der ersten medizinischen Bildgebungsvorrichtung (1) erzeugtes Bild beschreiben, in den Lernalgorithmus, wobei

erste Ausrichtungsdaten der Bildgebungsvorrichtung durch Tracking der ersten medizinischen Bildgebungsvorrichtung bestimmt werden; und

zweite Ausrichtungsdaten des zweiten Bildgebungsgeräts durch Tracking der zweiten medizinischen Bildgebungsvorrichtung ermittelt werden,

**dadurch gekennzeichnet, dass** die Steuerparameterdaten der zweiten Bildgebungsvorrichtung auf der Grundlage der ersten Bildgebungsvorrichtungsausrichtungsdaten und der zweite Bildgebungsvorrichtungsausrichtungsdaten so bestimmt werden, dass die Ausrichtung der zweiten medizinischen Bildgebungsvorrichtung (2) der Ausrichtung der ersten medizinischen Bildgebungsvorrichtung (1) entspricht; und durch Bestimmen (S22) von Parameterbeziehungsdaten durch Minimierung einer Verlustfunktion.

6. Computerimplementiertes Verfahren zur Bestimmung eines Steuerparameters zur Steuerung einer medizinischen Bildgebungsvorrichtung, wobei das Verfahren die folgenden Schritte umfasst:

   a) erste Bildgebungsgeräte-Betriebsparameterdaten werden erfasst (S31), die einen Wert eines Betriebsparameters einer ersten medizinischen Bildgebungsvorrichtung (1) beschreiben, wobei der Betriebsparameter eine Ausrichtung des ersten medizinischen Bildgebungsgeräts (1) relativ zu einer anatomischen Struktur in einem Navigationsbezugsystem ist;
   b) Bildsignaldaten werden erfasst (S32), die ein von der ersten medizinischen Bildgebungsvorrichtung (1) erzeugtes Bild beschreiben;
   c) zweite Bildgebungsvorrichtungssteuerparameterdaten, die einen Wert des Steuerparameters einer zweiten medizinischen Bildgebungsvorrichtung (2) beschreiben, werden bestimmt (S33), wobei der Steuerparameter der zweiten medizinischen Bildgebungsvorrichtung (2) eine Ausrichtung der zweiten medizinischen Bildgebungsvorrichtung (2) relativ zu der anatomischen Struktur in dem Navigationsbezugsystem ist, **dadurch gekennzeichnet, dass** der zweite Bildgebungsvorrichtungssteuerparameter so bestimmt wird, dass die Ausrichtung der zweiten medizinischen Bildgebungsvorrichtung (2) der Ausrichtung der ersten medizinischen Bildgebungsvorrichtung (1) entspricht, indem die ersten Bildgebungsvorrichtungsbetriebsparameterdaten und die Bildsignaldaten in einen lernenden Algorithmus eingegeben werden, der so konfiguriert ist, dass er eine Beziehung zwischen dem Wert des Betriebsparameters und dem Wert des Steuerparameters herstellt, der beispielsweise durch Ausführen des Verfahrens nach dem vorhergehenden Anspruch trainiert worden ist, und eine resultierende Verlustfunktion bestimmt wird.

7. Verfahren nach einem der vorhergehenden Ansprüche, wobei

   die Ausrichtung der ersten medizinischen Bildgebungsvorrichtung (1) eine Abbildungsperspektive der ersten medizinischen Bildgebungsvorrichtung (1) auf eine anatomische Struktur (3) definiert und
   die Ausrichtung der zweiten medizinischen Bildgebungsvorrichtung (2) eine Abbildungsperspektive der zweiten medizinischen Bildgebungsvorrichtung (2) auf die anatomische Struktur (3) definiert.

8. Verfahren nach einem der vorhergehenden Ansprüche, wobei

   das erste medizinische Bildgebungsgerät (1) ein Ultraschallbildgebungsgerät ist und
   das zweite medizinische Bildgebungsgerät (2) ein Röntgenbildgebungsgerät, z. B. ein C-Bogen, ist.

9. Programm, das, wenn es auf einem Computer (10) läuft oder auf einen Computer (10) geladen wird, den Computer (10) veranlasst, die Verfahrensschritte des Verfahrens nach einem der vorhergehenden Ansprüche durchzuführen.

10. Programmspeichermedium (11), auf dem das Programm nach dem vorhergehenden Anspruch gespeichert ist, oder ein Programmspeichermedium (11), auf dem Daten gespeichert sind, die die Modellparameter und die Architektur eines Lernalgorithmus definieren, der durch Ausführen des Verfahrens nach Anspruch 5 trainiert worden ist.

11. Datenträgersignal, das das Programm nach Anspruch 9 trägt, und/oder ein Datenträgersignal, das Daten trägt, die die Modellparameter und die Architektur eines Lernalgorithmus definieren, der durch Ausführen des Verfahrens nach Anspruch 5 trainiert worden ist.

12. Mindestens ein Computer (10) mit mindestens einem Prozessor und einem Speicher, wobei das Programm nach Anspruch 9 auf dem mindestens einen Prozessor läuft oder in den Speicher des Computers (10) geladen wird.

13. System (9) zur Bestimmung eines Steuerparameters zur Steuerung einer medizinischen Bildgebungsvorrichtung, umfassend:

   a) den mindestens einen Computer (10) nach dem vorhergehenden Anspruch;
   b) mindestens eine elektronische Datenspeichervorrichtung (12), die die Parameterbeziehungsdaten speichert; und

c) das Programmspeichermedium (11) nach Anspruch 10,

wobei der mindestens eine Computer (10) funktionsfähig gekoppelt ist mit

- die mindestens eine elektronische Datenspeichervorrichtung (12) zum Erfassen, von der mindestens einen elektronischen Datenspeichervorrichtung (12), der ersten Bildgebungsvorrichtungsbetriebsparameterdaten und zum Speichern, in der mindestens einen elektronischen Datenspeichervorrichtung (12), mindestens der zweiten Bildgebungsvorrichtungssteuerparameterdaten; und
- soweit das Programm den Computer (10) veranlasst, die Verfahrensschritte des Verfahrens nach Anspruch 6 durchzuführen, das Programmspeichermedium (11), um von dem Programmspeichermedium (11) Daten zu erfassen, die Modellparameter und eine Architektur des Lernalgorithmus definieren, der konfiguriert ist, eine Beziehung zwischen dem Wert des Betriebsparameters und dem Wert des Steuerparameters herzustellen.

**14.** System zur Steuerung einer medizinischen Bildgebungsvorrichtung, umfassend:

a) das System (9) nach dem vorhergehenden Anspruch;
b) die erste medizinische Bildgebungsvorrichtung (1); und
c) die zweite medizinische Bildgebungsvorrichtung (2),

wobei der mindestens eine Computer (10) funktionsfähig gekoppelt ist mit

- der ersten medizinischen Bildgebungsvorrichtung (1), um von der ersten medizinischen Bildgebungsvorrichtung (1) die ersten Bildgebungsvorrichtungsbetriebsdaten zu erfassen; und
- der zweiten medizinischen Bildgebungsvorrichtung (2), um an die zweite medizinische Bildgebungsvorrichtung (2) ein Steuersignal zur Steuerung der zweiten medizinischen Bildgebungsvorrichtung gemäß den zweiten Bildgebungsvorrichtungssteuerdaten auszugeben.

## Revendications

**1.** Procédé mis en œuvre par ordinateur pour déterminer un paramètre de commande pour commander un dispositif d'imagerie médicale, le procédé comprenant les étapes suivantes :

a) des données de paramètre de fonctionnement de premier dispositif d'imagerie sont acquises (S11) qui décrivent une valeur d'un paramètre de fonctionnement d'un premier dispositif d'imagerie médicale (1), dans lequel le paramètre de fonctionnement est une orientation du premier dispositif d'imagerie médicale (1) par rapport à une structure anatomique dans un système de référence de navigation ;
b) des données de relation de paramètre sont acquises (S12) qui décrivent une relation entre la valeur du paramètre de fonctionnement du premier dispositif d'imagerie médicale (1) et une valeur d'un paramètre de commande d'un deuxième dispositif d'imagerie médicale (2), dans lequel le paramètre de commande du deuxième dispositif d'imagerie médicale (2) est une orientation du deuxième dispositif d'imagerie médicale (2) par rapport à la structure anatomique dans le système de référence de navigation ;
c) des données de paramètre de commande de deuxième dispositif d'imagerie sont déterminées (S13) sur la base des données de paramètre de fonctionnement de premier dispositif d'imagerie et des données de relation de paramètre, dans lequel les données de paramètre de commande de deuxième dispositif d'imagerie décrivent la valeur du paramètre de commande du deuxième dispositif d'imagerie médicale (2) ;

des données d'orientation de premier dispositif d'imagerie sont déterminées par suivi du premier dispositif d'imagerie médicale ; et
des données d'orientation de deuxième dispositif d'imagerie sont déterminées par suivi du deuxième dispositif d'imagerie médicale,
**caractérisé en ce que** les données de paramètre de commande de deuxième dispositif d'imagerie sont déterminées sur la base des données d'orientation de premier dispositif d'imagerie et des données d'orientation de deuxième dispositif d'imagerie de sorte que l'orientation du deuxième dispositif d'imagerie médicale (2) correspond à l'orientation du premier dispositif d'imagerie médicale (1).

**2.** Procédé selon la revendication précédente, dans lequel le paramètre de fonctionnement du premier dispositif d'imagerie médicale (1) comprend une pluralité de paramètres de fonctionnement et le paramètre de commande du deuxième dispositif d'imagerie médicale (2) comprend une pluralité de paramètres de commande et les données de relation de paramètre sont ou ont été déterminées par détermination d'une matrice de relation de paramètre résultant de la solution, par exemple par application d'un procédé de décomposition de valeur singulière, d'un système d'équations linéaires reliant un vecteur comprenant des entrées représentant la pluralité de paramètres de fonctionnement à un vecteur comprenant des entrées représentant la pluralité de paramètres de commande.

3. Procédé selon la revendication 1, dans lequel le paramètre de fonctionnement du premier dispositif d'imagerie médicale (1) comprend une pluralité de paramètres de fonctionnement et le paramètre de commande du deuxième dispositif d'imagerie médicale (2) comprend une pluralité de paramètres de commande et les données de relation de paramètre sont ou ont été déterminées en fourniture en entrée de valeurs de la pluralité de paramètres de fonctionnement et de valeurs de la pluralité de paramètres de commande et de données de signal d'imagerie décrivant une image générée par le premier dispositif d'imagerie médicale à un algorithme d'apprentissage configuré pour établir une relation entre les valeurs de la pluralité de paramètres de fonctionnement et les valeurs de la pluralité de paramètres de commande.

4. Procédé selon la revendication précédente, dans lequel l'algorithme d'apprentissage est ou a été configuré pour établir la relation entre les valeurs de la pluralité de paramètres de fonctionnement et les valeurs de la pluralité de paramètres de commande par fourniture en entrée, à l'algorithme d'apprentissage, de valeurs de la pluralité de paramètres de fonctionnement et de valeurs de la pluralité de paramètres de commande et de données de signal d'imagerie décrivant une image générée par le premier dispositif d'imagerie médicale (1) et détermination d'une fonction de perte résultante.

5. Procédé mis en œuvre par ordinateur pour entraîner un algorithme d'apprentissage afin d'établir une relation entre les valeurs d'une pluralité de paramètres de fonctionnement d'un premier dispositif d'imagerie médicale (1), dans lequel le paramètre de fonctionnement est une orientation du premier dispositif d'imagerie médicale (1) par rapport à une structure anatomique dans un système de référence de navigation et des valeurs d'une pluralité de paramètres de commande d'un deuxième dispositif d'imagerie médicale (2), dans lequel le paramètre de commande du deuxième dispositif d'imagerie médicale (2) est une orientation du deuxième dispositif d'imagerie médicale (2) par rapport à la structure anatomique dans le système de référence de navigation, le procédé comprenant :

la fourniture en entrée (S21), à l'algorithme d'apprentissage, de données de paramètre de fonctionnement de premier dispositif d'imagerie décrivant des valeurs de la pluralité de paramètres de fonctionnement et de données de paramètre de commande du deuxième dispositif d'imagerie décrivant des valeurs de la pluralité de paramètres de commande et de données décrivant une image générée par le premier dispositif d'imagerie médicale (1), dans lequel
des données d'orientation de premier dispositif d'imagerie sont déterminées par suivi du premier dispositif d'imagerie médicale ; et
des données d'orientation de deuxième dispositif d'imagerie sont déterminées par suivi du deuxième dispositif d'imagerie médicale,
**caractérisé en ce que** les données de paramètre de commande de deuxième dispositif d'imagerie sont déterminées sur la base des données d'orientation de premier dispositif d'imagerie et des données d'orientation de deuxième dispositif d'imagerie de sorte que l'orientation du deuxième dispositif d'imagerie médicale (2) correspond à l'orientation du premier dispositif d'imagerie médicale (1) ; et par
la détermination (S22) de données de relation de paramètre par minimisation d'une fonction de perte.

6. Procédé mis en œuvre par ordinateur pour déterminer un paramètre de commande pour commander un dispositif d'imagerie médicale, le procédé comprenant les étapes suivantes :

a) des données de paramètre de fonctionnement de premier dispositif d'imagerie sont acquises (S31) qui décrivent une valeur d'un paramètre de fonctionnement d'un premier dispositif d'imagerie médicale (1), dans lequel le paramètre de fonctionnement est une orientation du premier dispositif d'imagerie médicale (1) par rapport à une structure anatomique dans un système de référence de navigation ;
b) des données de signal d'imagerie sont acquises (S32) qui représentent une image générée par le premier dispositif d'imagerie médicale (1) ;
c) des données de paramètre de commande de deuxième dispositif d'imagerie décrivant une valeur du paramètre de commande d'un deuxième dispositif d'imagerie médicale (2) sont déterminées (S33), dans lequel le paramètre de commande du deuxième dispositif d'imagerie médicale (2) est une orientation du deuxième dispositif d'imagerie médicale (2) par rapport à la structure anatomique dans le système de référence de navigation, **caractérisé en ce que** le paramètre de commande de deuxième dispositif d'imagerie est déterminé de sorte que l'orientation du deuxième dispositif d'imagerie médicale (2) correspond à l'orientation du premier dispositif d'imagerie médicale (1) par fourniture en entrée, à un algorithme d'apprentissage configuré pour établir une relation entre la valeur du paramètre de fonctionnement et la valeur du paramètre de commande qui a été entraîné par exemple par exécution du procédé selon la revendication précédente, des données de paramètre de fonctionnement de premier dispositif d'imagerie et des données de signal d'imagerie et détermination d'une fonction de perte résultante.

7. Procédé selon l'une quelconque des revendications précédentes, dans lequel

l'orientation du premier dispositif d'imagerie médicale (1) définit une perspective d'imagerie du premier dispositif d'imagerie médicale (1) sur une structure anatomique (3) et
l'orientation du deuxième dispositif d'imagerie médicale (2) définit une perspective d'imagerie du deuxième dispositif d'imagerie médicale (2) sur la structure anatomique (3).

8. Procédé selon l'une quelconque des revendications précédentes, dans lequel

le premier dispositif d'imagerie médicale (1) est un dispositif d'imagerie à ultrasons et
le deuxième dispositif d'imagerie médicale (2) est un dispositif d'imagerie à rayons X, par exemple un arceau.

9. Programme qui, lorsqu'il s'exécute sur un ordinateur (10) ou lorsqu'il est chargé sur un ordinateur (10), amène l'ordinateur (10) à réaliser les étapes de procédé du procédé selon l'une quelconque des revendications précédentes.

10. Support (11) de stockage de programme sur lequel est stocké le programme selon la revendication précédente ou support (11) de stockage de programme sur lequel sont stockées des données définissant les paramètres de modèle et l'architecture d'un algorithme d'apprentissage qui a été entraîné par l'exécution du procédé selon la revendication 5.

11. Signal porteur de données portant le programme selon la revendication 9, et/ou un signal porteur de données portant des données définissant les paramètres de modèle et l'architecture d'un algorithme d'apprentissage qui a été entraîné par l'exécution du procédé selon la revendication 5.

12. Au moins un ordinateur (10) comprenant au moins un processeur et une mémoire, dans lequel le programme selon la revendication 9 s'exécute sur l'au moins un processeur ou est chargé dans la mémoire de l'ordinateur (10).

13. Système (9) permettant de déterminer un paramètre de commande pour commander un dispositif d'imagerie médicale, comprenant :

a) l'au moins un ordinateur (10) selon la revendication précédente ;
b) au moins un dispositif électronique de stockage de données (12) stockant les données de relation de paramètre ; et
c) le support (11) de stockage de programme selon la revendication 10,

dans lequel l'au moins un ordinateur (10) est couplé de manière fonctionnelle

- à l'au moins un dispositif électronique de stockage de données (12) pour acquérir, à partir de l'au moins un dispositif électronique de stockage de données (12), les données de paramètre de fonctionnement de premier dispositif d'imagerie, et pour stocker, dans l'au moins un dispositif électronique de stockage de données (12), au moins les données de paramètre de commande de deuxième dispositif d'imagerie ; et
- dans la mesure où le programme amène l'ordinateur (10) à réaliser les étapes de procédé du procédé selon la revendication 6, au support de stockage (11) de programme pour acquérir, à partir du support (11) de stockage de programme, des données définissant des paramètres de modèle et une architecture de l'algorithme d'apprentissage configuré pour établir une relation entre la valeur du paramètre de fonctionnement et la valeur du paramètre de commande.

14. Système permettant de commander un dispositif d'imagerie médicale, comprenant :

a) le système (9) selon la revendication précédente ;

b) le premier dispositif d'imagerie médicale (1) ; et
c) le deuxième dispositif d'imagerie médicale (2),

dans lequel l'au moins un ordinateur (10) est couplé de manière fonctionnelle

- au premier dispositif d'imagerie médicale (1) pour acquérir, à partir du premier dispositif d'imagerie médicale (1), les données de fonctionnement de premier dispositif d'imagerie ; et
- au deuxième dispositif d'imagerie médicale (2) pour émettre, vers le deuxième dispositif d'imagerie médicale (2), un signal de commande pour commander le deuxième dispositif d'imagerie médicale selon les données de commande de deuxième dispositif d'imagerie.

```
┌─────────────────────────────────┐
│              S11                │
│    acquire first imaging device │
│     operating parameter data    │
└─────────────────────────────────┘
                 │
                 ▼
┌─────────────────────────────────┐
│              S12                │
│   acquire parameter relationship│
│              data               │
└─────────────────────────────────┘
                 │
                 ▼
┌─────────────────────────────────┐
│              S13                │
│     determine second imaging    │
│   device control parameter data │
└─────────────────────────────────┘
```

**Fig. 1**

S21
input first imaging device
operating parameter and
second imaging device control
parameter data

S22
determine parameter
relationship data

**Fig. 2**

```
┌─────────────────────────────────────┐
│               S31                   │
│     acquire first imaging device    │
│      operating parameter data       │
└─────────────────────────────────────┘
                  │
                  ▼
┌─────────────────────────────────────┐
│               S32                   │
│      acquire imaging signal data    │
│                                     │
└─────────────────────────────────────┘
                  │
                  ▼
┌─────────────────────────────────────┐
│               S33                   │
│       determine second imaging      │
│     device control parameter data   │
└─────────────────────────────────────┘
```

**Fig. 3**

**Fig. 4**

**Fig. 5**

**Fig. 6**

# Training

**Fig. 7**

Prediction

Fig. 8

9

**Fig. 9**

**EP 4 352 741 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180235573 A1
- JP 6014866 B
- WO 2019234495 A
- US 20200281556 A1